(19) Europäisches Patentamt
European Patent Office
Office européen des brevets

(11) Publication number: **0 356 894**
**A1**

# (12) EUROPEAN PATENT APPLICATION

(21) Application number: 89115519.4

(22) Date of filing: 23.08.89

(51) Int. Cl.5: C07K 9/00 , C12P 21/04 , A61K 37/02 , //(C12P21/04, C12R1:04)

Claims for the following Contracting States: ES + GR.

(30) Priority: 27.08.88 EP 88114022

(43) Date of publication of application: 07.03.90 Bulletin 90/10

(84) Designated Contracting States: AT BE CH DE ES FR GB GR IT LI LU NL SE

(71) Applicant: HOECHST AKTIENGESELLSCHAFT
Postfach 80 03 20
D-6230 Frankfurt am Main 80(DE)

(72) Inventor: Franco, Christopher Milton Mathew, Dr.
74A, "The Westminister" N.S. Manikikar Marg
Sion Bombay 400 022(IN)
Inventor: Chatterjee, Sugata, Dr.
H1/2 Hoechst Quarters Darga Road
Mulund(West) Bombay 400 082(IN)
Inventor: Vijakumar, Erra Koteswara Satya, Dr.
K-3 Hoechst Quarters Darga Road
Mulund(West) Bombay 400 082(IN)
Inventor: Chatterjee, Deepak Kumar, Dr.
Sheetal Bungalow No,2 Mahatma Phule Road
Mulund(East) Bombay 400 081(IN)
Inventor: Ganguli, Bimal Naresh, Dr.
"Saurayuth" 173 Central Avenue
Chembur Bombay 400 071(IN)
Inventor: Rupp, Richard Helmut, Dr.
Roederweg 16a
D-6240 Königstein/Taunus(DE)
Inventor: Fehlhaber, Hans-Wolfram, Dr.
Thomas-Mann-Strasse 5a
D-6270 Idstein/Taunus(DE)
Inventor: Kogler, Herbert, Dr.
Breslauer Strasse 39
D-6233 Kelkheim(Taunus)(DE)
Inventor: Seibert, Gerhard, Prof.Dr.
Gläserweg 21
D-6100 Darmstadt(DE)
Inventor: Teetz, Volker, Dr.
An der Tann 20
D-6238 Hofheim am Taunus(DE)

## (54) A novel glycopeptide antibiotic Decaplanin, a process for its production and its use as medicament.

(57) Decaplanin, a compound of the formula

EP 0 356 894 A1

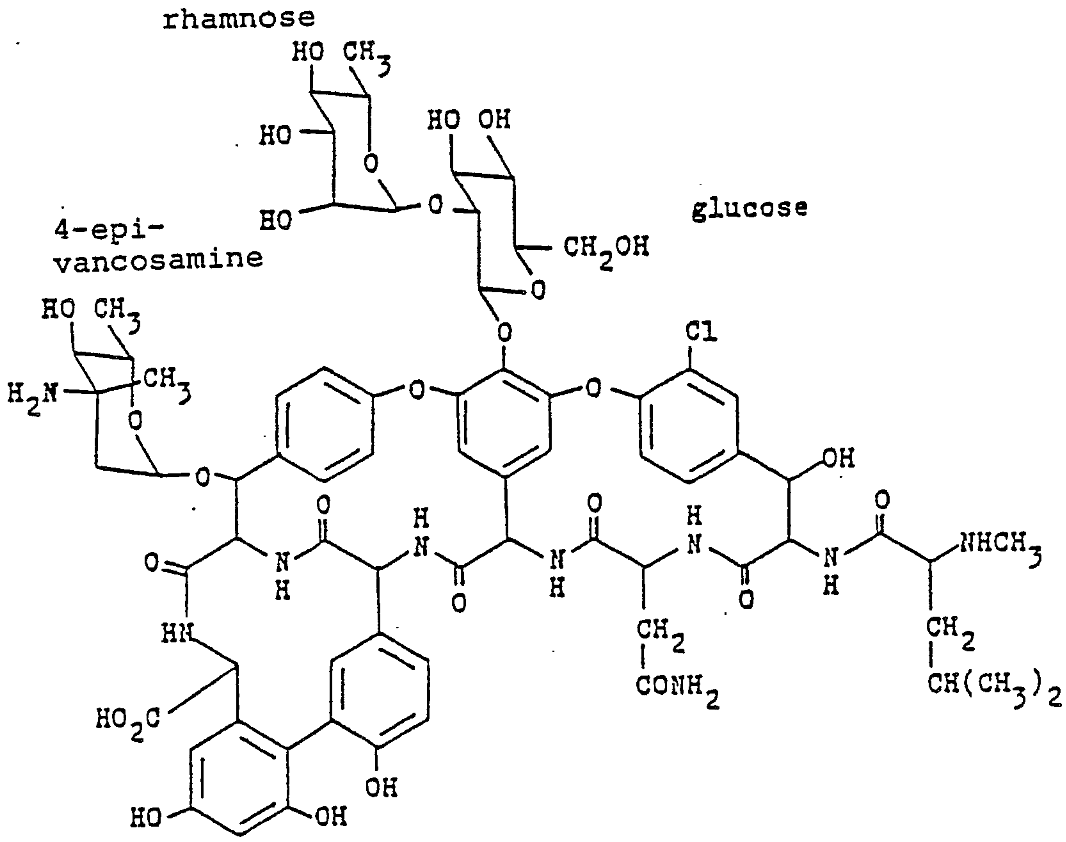

has an antibiotic activity.

# A novel glycopeptide antibiotic, Decaplanin, a process for its production and its use as medicament

This invention relates to a novel glycopeptide antibiotic, decaplanin, a process for its production by means of an actinomycete culture Y-86,36910 (deposited with the Deutsche Sammlung für Mikroorganismen according to the Treaty of Budapest at August 5, 1988 with the number DSM 4763) its variants and mutants and its use as medicament.

Actinomycete culture number HIL Y-86,36910 (actinomycete culture No. Y-86,36910) was isolated from soil collected near Pune, Maharashtra, India. Variants and mutants of actinomycete culture number HIL Y-86,36910 can be obtained in a known manner using a mutagen, such as ultraviolet light or N.methyl-N′-nitro-N′-nitrosoguanidine (c.f. for example "Allgemeine Mikroobiologie", H.G. Schlegel, Georg Thieme Verlag Stuttgart, New York, 1985). Actinomycete culture No. Y-86,36910 belongs to the order Actinomycetales and the genus Kibdelosporangium. It is considered to be a new strain since it differs from known strains in many of its morphological, cultural and physiological characteristics as will be clear from the description hereinafter. It is considered to be a new strain also because it produces a new antibiotic herein called Decaplanin, as will be clear from the description hereinafter.

Decaplanin, a compound of the formula IV is a glycopeptide antibiotic of the vancomycin type. The glycopeptide antibiotics of the vancomycin-type described in the literature are Antibiotic A 477 in US patent 3,780,174 of December 18, 1972. Antibiotic A 35512 in J. Antibiotics 33, 1407-1416, 1980; Antibiotic A 41030 and Antibiotic A 47934 in the Programme and Abstracts of the 23rd Interscience Conference on Antimicrobial Agents and Chemotherapy, Abstract Nos. 440 and 443 respectively, p. 164, Las Vegas, 1983; Antibiotic AAJ-271 in the Programme and Abstracts of the 26th Interscience Conference on Antimicrobial Agents and Chemotherapy, Abstract No. 226, p. 137, New Orleans, 1986; Antibiotic AB-65 in J. Antibiotics 28, 395-397, 1975; Actaplanins in J. Antibiotics, 37, 85-95, 1984; Actinoidins in Tetrahedron Lett., 31, 2861-2864; 1979; Actinoidin $A_2$ in J. Antibiotics 40, 165-172, 1987; Aridicins in J. Antibiotics 38 555-560 and 561-571, 1985; Avoparicin in J. Am. Chem. Soc. 101, 2237-2239, 1980 and 102 1671-1684, 1980; Chloropolysporins in 5 consecutive papers in J. Antibiotics 40, 917-952, 1987; Eremomycin in Antibiotik. Med. Biotekhnol. 32, 571-576, 1987; Kibdelins in J. Antibiotics 39, 1386-1406, 1986; Antibiotic LL-AM-374 in US Patent 3,803,306 of April 9, 1974; Antibiotic LL-AV-290 in Antimicrob. Agents and Chemother. 1968, 242-245. Mannopeptins in J. Antibiotics 28, 503-507, 1975. Antibiotic OA-7653 in Agric. Biol. Chem. 47, 499-506, 1983; and J. Org. Chem. 53, 471-477, 1988; Orienticins in Chem. Abstr., 108, 164466f, 1988; Parvodicin in J. Antibiotics 40, 970-990, 1987; Ristocetins in J. Chem. Soc. Chem. Commun. 1979; 906-908, 1979; and in Antibiotics Annual 1957-1957, Eds. H. Welch & F. Marti-Ibanez, pp. 699-705, Medical Encyclopedia, Inc. New York, 1957; Ristomycins A and B in Antibiotiki 14, 594-597 (1969); Teicoplanins (teichomycins) in J. Antibiotics 31, 170-177, 1978 and in J. Am. Chem. Soc. 106, 4891-4895 and 4895-4902, 1984 and Vancomycin in J. Am. Chem. Soc. 105, 6915-6922, 1983.

Review articles of glycopeptide antibiotics are also found in Ann. Rev. Microbiol. 38, 339-357, 1984; Topics in Antibiotic Chemistry 5, 119-158, 1980; "CRC Handbook of Antibiotic Compounds" Volume I, pp. 305.323, Naros Berdy (author), CRC Press, Boca Raton, Florida, 1980.

Other summarizing descriptions of the glycopeptide antibiotics of the vancomycin type may be referred to in the "Index of Antibiotics from Antinomycetes", Hamao Umezawa, Editor in Chief, Volume I, Pages 568-570, 675, 733, Univ. Park Press, State College, Pennsylvania, U.S.A., 1967; and volume 11, pages 105, 106, 167, 625, 1081, 1100, Univ. Park Press, Baltimore, USA, 1978.

Glycopeptide antibiotics of the vancomycin type are characterised by the presence of a heptapeptidic aglycone as shown in Formula I

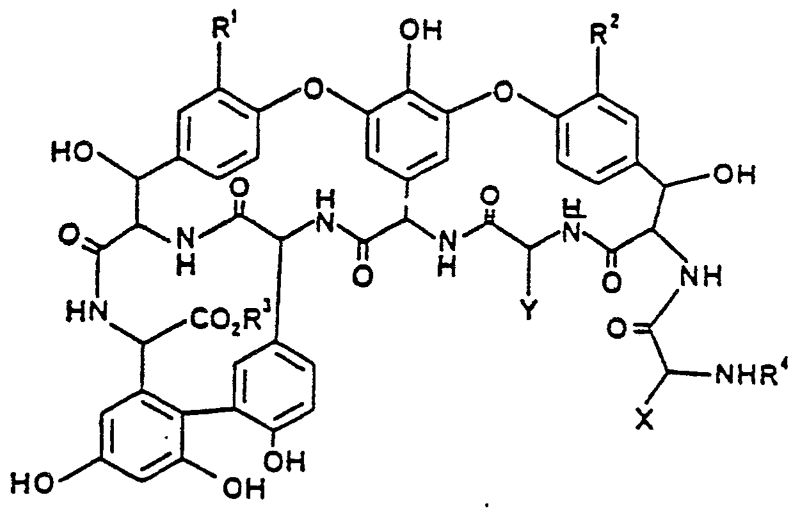

(I)

wherein $R^1$, $R^2$ = H and/or Cl, $R^3$, $R^4$ = H and/or alkyl group; X, Y components can be either two different amino acids or constituents of the same amino acid; Sugars (basic and neutral) can be attached to the benzylic and/or the phenolic OH groups. Most of these antibiotics also contain one or more Cl atoms in the vancomycinic acid component and/or in the X, Y component. The aglycone described above contains cross-linked aromatic acids like vancomycinic acid (Formula II, wherein $R^1$, $R^2$ = H and/or Cl) and actinoidinic acid (Formula III).

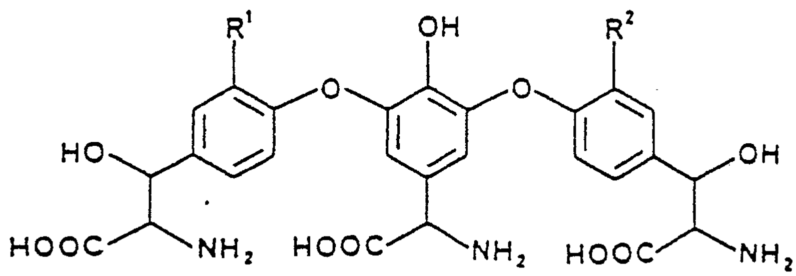

(II)

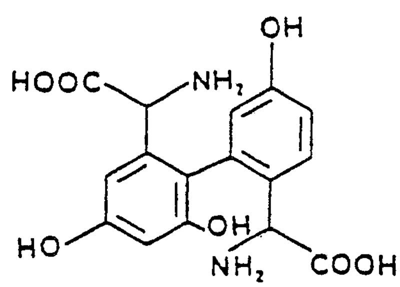

(III)

However, from all the data available the antibiotic Decaplanin is clearly different from any glycopeptide antibiotic of the vancomycin type described in the above quoted literature. Furthermore, Chemical Abstract Service Online Literature search performed with the search keys of molecular weight and molecular formula also confirmed the fact that Decaplanin is a new compound.

The structure of Decaplanin is shown in Formula IV

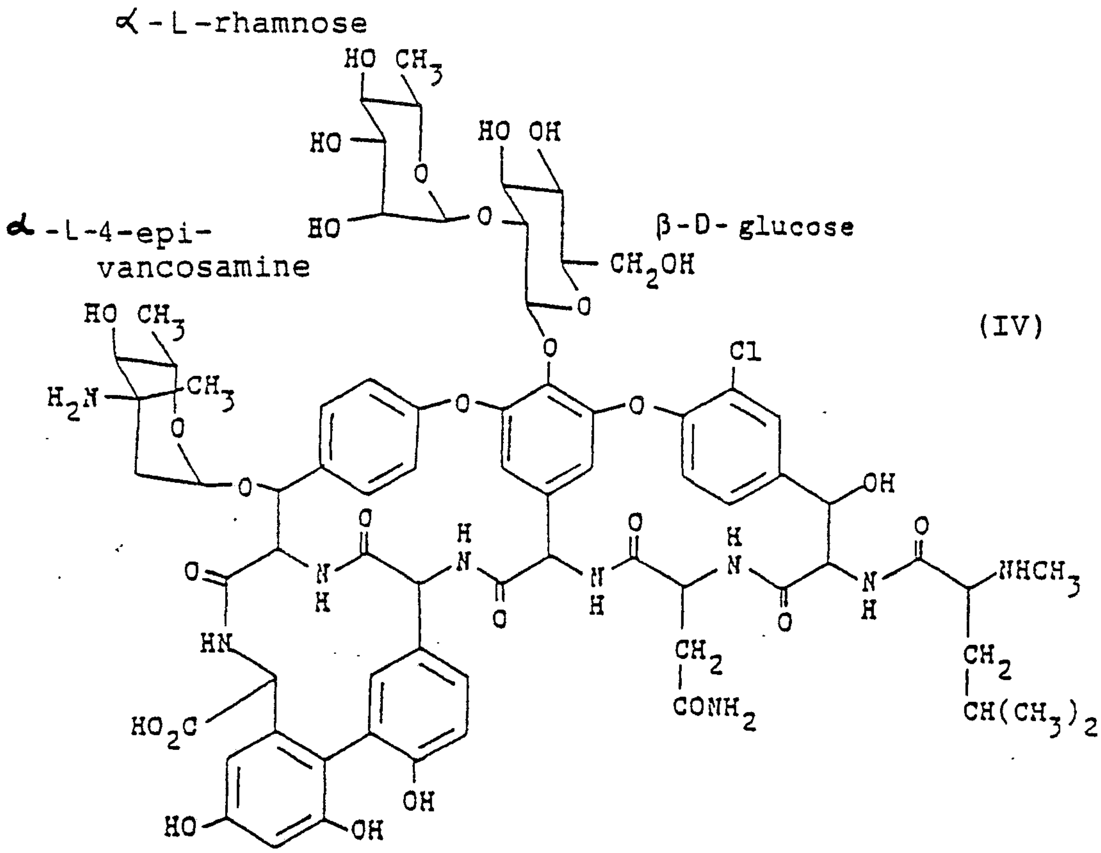


(IV)

Glycopeptide antibiotics of the vancomycin type are of importance in medicine as antimicrobial agents against a wide spectrum of Grampositive bacteria including penicillin-, methicillin-, and oxacillin-resistant strains of Staphylococcus and Streptococcus sp.

The novel antibiotic of this invention is active against a number of pathogenic microorganisms and accordingly, finds utility in human and veterinary medicine. More precisely, Decaplanin can be used as a therapeutic drug for the treatment of infectious deseases caused by Gram-positive bacteria such as, for example, Staphylococcus aureus, Staphylococcus epidermidis, Streptococcus pyogenes, Streptococcus viridans, Streptococcus faecalis, Diplococcus pneumoniae, Bacteroides fragilis, Clostridium difficile, Corynebacterium sp., Actinobacter sp. and Fusobacterium sp.

Decaplanin may also be used as a feed additive for poultry and livestock as a growth performance promoter.

A further object of the present invention is to provide a process for the production of the new antibiotic Decaplanin from culture No. Y-86,36910, its mutants and variants. The said process comprises cultivation of culture No. Y-86,36910, its variants and mutants, under aerobic conditions at a temperature between 18 °C and 40 °C, in an aqueous nutrient medium containing sources of carbon, sources of nitrogen, together with essential nutrient organic and mineral salts, at a pH between 5.5 and 8.5, until the medium exhibits a substantial antibiotic activity, and isolation and purification of the said antibiotic from the culture broth.

According to the present invention there is also provided a process for the isolation of culture No. Y-86,36910 from soil using a nutrient medium at a pH of 6,5 to 8,5 in a known manner.

The nutrient medium used for isolation of the microorganism from soil consists of carbon and nitrogen sources, inorganic nutrient salts and solidifying agents. Sources of carbon may be glucose, starch, dextrin, glycerol, sucrose or molasses. Sources of nitrogen may be peptone, yeast extract, beef extract, malt extract, casein or amino acids such as arginine or asparagine. The solidifying agent may, for example, be agar. The inorganic nutrient salts may be for example salts of sodium, potassium, magnesium, calcium, phosphorous or sulphur. As trace elements salts of iron, copper, manganese, zinc and cobalt may be incorporated into the medium.

The microorganism of this invention is a Gram-positive, non-acid-fast, filamentous organism that forms aerial mycelia from branched substrate mycelia. The well-developed substrate mycelium may exhibit varying degrees of fragmentation without hyphal displacement. In shake flasks and fermentors fragmentation is usually observed. The aerial mycelium produced straight or slightly curved are of rodshaped, smooth

walled spores. The spores have a dimension of 0.2-0.5 x 1-3 µm. The spore chains are usually very long with more than 50 spores per chain, but a few short chains of 10 spores or less are also present. Sporangium-like structures are also observed. These sporangium-like structures, analogous to the conidial structures seen in Actinoplanaceae, are surrounded by well-defined walls but do not contain spores. Despite extensive manipulation and experimentation, development or release of spores from these sporangium-like structures is not observed. They germinate directly, producing one or more germ tubes. Similar morphological features are also described in the genus Kibdelosporangium as reported in Int. J. System. Bacterol. 36, 47-54, 1986. The surface of the sporangium-like bodies seen on ISP (International Streptomyces Project) media 2, 3, 4, 5 and 7 and on nutrient agar, are rough and wrinkled and their size ranges from 2-16 µm.

On some media actinomycete culture No. Y-86,36910 produces characteristic crystals in the agar.

The cultural characteristics of the microorganism on various agar media are described as follows

| 1. Yeast extract - malt extract agar | |
|---|---|
| (ISP medium 2) | |
| Growth : | good, dry, wrinkled |
| Aerial mycelium : | moderate, powdery, yellowish-white |
| Reverse : | yellow-brown |
| Soluble pigment : | brownish green |

| 2. Oatmeal agar | |
|---|---|
| (ISP medium 3) | |
| Growth : | moderate, centre-elevated, dry |
| Aerial mycelium : | moderate, powdery, yellowish-white |
| Reverse : | pale yellow |
| Soluble pigment : | none |

| 3. Inorganic salts - starch agar | |
|---|---|
| (ISP medium 4) | |
| Growth : | moderate, centre elevated, dry |
| Aerial mycelium : | weak, powdery, yellowish-white |
| Reverse : | pale yellow |
| Soluble pigment : | none |

| 4. Glycerol - asparagine agar | |
|---|---|
| (ISP medium 5) | |
| Growth : | moderate, elevated, dry |
| Aerial mycelium : | weak, powdery, yellowish white |
| Reverse : | yellow |
| Soluble pigment : | none |

| 5. Peptone - yeast extract - iron agar | |
|---|---|
| (ISP medium 6) | |
| Growth : | good, elevated, moist |
| Aerial mycelium : | none |
| Reverse : | pale yellow |
| Soluble pigment : | none |

| 6. Tyrosine agar | |
|---|---|
| (ISP medium 7) | |
| Growth : | good, elevated, dry |
| Aerial mycelium : | moderate, powdery, yellowish white |
| Reverse : | pale yellow |
| Soluble pigment : | none |

| 7. Socrose - nitrate agar | |
|---|---|
| (Czapek's agar) | |
| Growth : | weak, raised, dry |
| Aerial mycelium : | none |
| Reverse : | pale yellow |
| Soluble pigment : | none |

| 8. Peptone - beef extract agar | |
|---|---|
| (Nutrient agar) | |
| Growth : | abundant, elevated |
| Aerial mycelium : | very scant, powdery, yellowish white |
| Reverse : | pale yellow |
| Soluble pigment : | none |

Reverse of colony: No distinctive pigments are produced on any of the media used. Substrate mycelium shows no response to change in pH.

Soluble pigment: Brownish green pigment observed on ISP medium 2 is not a pH indicator.

The optimum growth range for the organism of this invention is from 25 °C to 35 °C with poor growth at 4 °C and no growth at 45 °C. This microorganism liquefies gelatin in glucose-peptone-gelatin medium, hydrolyzes starch in starch-inorganic salts agar and reduces nitrate in nitrate broth.

No melanin production is observed in tyrosine agar, peptone-yeast extract-iron agar or tryptone-yeast extract broth. This microorganism shows no cellulolytic activity, does not coagulate milk, shows a negative tyrosinase reaction and negative hydrogen sulfide production and only weakly peptonizes milk.

On Czapek's solution agar the growth is good with flat, smooth, white colonies.

The carbon assimilation pattern of this microorganism is as follows (in Pridham-Gottlieb's medium):

Positive: D-glucose, L-arabinose, D-xylose, M-Inositol, D-mannitol, D-fructose, galactose, salicin, maltose, cellobiose, mannose, lactose and sodium glutamate.

Doubtful: Sucrose, raffinose.

Negative: Rhamnose, cellulose and dulcitol.

Actinomycete culture No. Y-86,36910 is inhibited by streptomycin at concentrations greater than 0.2 mg/ml, can tolerate NaCl at concentrations between 5-7 % and has a pH tolerance range of 5.5-10.5.

Cell wall analysis of actinomycete culture number Y-86,36910 shows the presence of type IV cell wall containing meso-diamino pimelic acid, D-glutamic acid, DL-alanine, muramic acid, N-acetyl-D-glucosamine, D-galactose and arabinose and a type A whole cell sugar pattern of D-galactose and arabinose. No mycolic acids are present.

Actinomycete culture No. HIL Y-86,36910 has chemotaxonomic properties, cultural characteristics and morphology that are consistent with its assignment to the genus Kibdelosporangium. A differentiating comparison with the three reported strains of Kibdelosporangium species, viz. Kibdelosporangium aridum ATCC 39323 (Int. J. Systemic Bacteriology 36, 47-54, 1986), K. aridum subsp. largum ATCC 39922 (J. Antibiotics 39, 1386-1394, 1986) and K. philippinense NRRL 18198 (Int. J. Systemic Bacteriology 38, 282-286, 1988), is shown below.

| | K. aridum ATCC 39323 | K. aridum subsp. largum ATCC 39922 | K. philippinense NRRL 18198 | Actinomycete culture No. Y-86,36910 |
|---|---|---|---|---|
| Reverse Color | Off white | Off white | Orange/ Yellow | Yellow |
| Soluble Pigment | Rare | Rare | Occasional | Occasional |
| Cell Wall: Rhamnose | + | + | - | - |

| | K. aridum ATCC 39323 | K. aridum subsp. largum ATCC 39922 | K. philippinense NRRL 18198 | Actinomycete culture No. Y-86,36910 |
|---|---|---|---|---|
| Utilization of: | | | | |
| Arabinose | + | + | - | + |
| Dextrin | + | + | - | + |
| Raffinose | + | + | - | ± |
| NaCl Tolerance (%) | 7 | 7 | 2 | 7 |
| $NO_3$ Redn. | - | - | + | + |
| Peptonization of Milk | + | + | - | - |
| Fatty Acid Analysis (%): | | | | |
| Iso-14:0 | 1.3- 2.8 | N.T. | 11 -13.4 | 2.1 |
| Iso-16:0 | 20.7-36.7 | N.T. | 63.9-67.5 | 7.1 |
| Anteiso-15:0 | 8.0-11.7 | N.T. | 1.9 | 5.1 |
| Trans-16:1 | 2.1- 4.1 | N.T. | absent | absent |
| 15:0 | 0.8 | N.T. | N.T. | 5.1 |
| Cis-17:1 | absent | N.T. | N.T. | 11.4 |
| 17:0 | 1.29 | N.T. | N.T. | 10.2 |
| Antibiotic Produced | Ardicins | Kibdelins | Ristocetin Type | Decaplanin |

N.T. = Not Tested

The published information on the cultural and physiological characteristics of the known microorganisms show clear differences when compared with the microorganism of this invention. In addition, when actinomycete culture No. Y-86,36910 is fermented it produces the novel glycopeptide antibiotic Decaplanin.

Therefore the microorganism of this invention is proposed as a new species of Kibdelosporangium and has been named Kibdelosporangium deccaensis sp. nov.

It may be well understood to those skilled in the art that this invention is not limited to the particular organism which has been specified above but includes all those spontaneous and artificial mutants and variants derived from the said microorganism which are capable of producing the new glycopeptide antibiotic Decaplanin.

According to the present invention there is provided a process for the production of a new antibiotic Decaplanin, said process comprising cultivating actinomycete culture No. Y-86,36910 by fermentation preferably at a pH between 6.0 and 8.0 and a temperature between 18-40 °C under aerobic conditions in a nutrient medium containing sources of carbon and nitrogen, nutrient inorganic salts and trace elements and isolating the compounds from the culture broth in a known manner such as herein described.

The carbon sources used in the nutrient medium for production of the novel antibiotics may be, for example, glucose, starch, dextrin, glycerol, sucrose, molasses or oil. Sources of nitrogen used in the nutrient medium for production of the novel antibiotics may be, for example, soyabean meal, yeast extract, beef extract, malt extract, cornsteep liquor, peptone, or casein. Nutrient inorganic salts/mineral salts used in the nutrient medium for production of the novel antibiotics may be, for example, sodium chloride, magnesium sulphate, potassium nitrate, ammonium sulphate or calcium carbonate. As trace elements salts of iron, manganese, copper, zinc, cobalt or other such heavy metals may be used.

In particular, actinomycete culture No. Y-86,36910 is cultivated at 29 °C (±1 °C) and pH 6.5. The fermentation is preferably stopped after 66-68 hours when maximum yields of the compound are obtained. The fermentation may, preferably, be a submerged fermentation. The process of fermentation and formation of the novel antibiotic complex can be monitored by the antibacterial activity of the culture fluid and mycelium against Staphyloccus aureus 209 P and Micrococcus luteus by the known agar plate diffusion method.

If desired, an antifoaming agent such as Desmophen® (a linear polyether based on propylene oxide - Bayer AG, Leverkusen, Germany) is used in the nutrient medium during fermentation of the culture.

Decaplanin can be obtained from the culture broth by direct adsorption on suitable adsorbents. For example, the culture filtrate containing the compound according to this invention can be adsorped on active charcoal, Diaion® HP 20 (high porosity adsorbent resin based on a polystyrene-divinylbenzene copolymer - Mitsubishi Chemical Industries, Japan) or Amberlite® XAD (porous adsorbent resin based on polystyrene or acrylic acid ester - Rohm & Haas Co., U.S.A.). The preferred adsorbent is Diaion® HP 20. The compound according to the invention can be eluted from the adsorbents using appropriate mobile phases such as methanol, acetonitrile or acetone, either used singly, in combination with each other or with water, and the eluates are preferably evaporated to dryness. The preferred eluents are aqueous methanol and acetone.

Decaplanin may also be isolated from the culture filtrate using ion-exchange chromatography. A suitable resin would be a cation-exchange resin of the weakly acidic, copolymer of methylacrylic acid and divinylbenzene, type such as Amberlite® IRC-50 (Rohm & Haas Co., U.S.A.). In this. procedure the culture filtrate is subjected to columns chromatography using the cation-exchange resin Amberlite® IRC-50 ($H^+$). The compound according to the invention is first adsorbed on the ion-exchanger and then eluted out using appropriate mobile phases such as aqueous or methanolic solutions of dilute hydrochloric acid; preferably a 0.1 N HCl solution in water is used. The active eluates thus obtained are pooled and concentrated. Amberlite® IRA-68 $Cl^-$, an anion-exchange resin of the weakly basic type may also be used to decolourize the active eluates.

The aforementioned concentrated active eluates containing Decaplanin can be further purified in a number of ways. For example, readsorption and elution processes with activated carbon, Amberlite® XAD-4 and 7, Diaion® HP-20, gel filtration with Sephadex® LH-20 and G series gels (gels of defined porosity on agarose - Pharmacia Fine Chemicals AB, Sweden) and its equivalents, and ion-exchange gel-filtration with Sephadex® DEAE (diethylaminoethyl) gels, can be conveniently combined for further purification. In addition, thin-layer chromatography, medium-pressure and high-pressure liquid chromatography using suitable adsorbents such as silica gel, alumina and modified silica gel-$C_{18}$ with suitable solvent systems may be used. Furthermore, counter current chromatography with a particular solvent system may work well for the said purpose. The preferred purification methods include repeated adsorption and elution using Diaion® HP-20 followed by medium-liquid chromatography using modified silica gel $C_{18}$ with methanol- or acetonitrile-aqueous salt solutions as eluting solvents.

Decaplanin can be transformed into its pharmacologically acceptable salts for example with inorganic and organic acids such as HCl, $H_2SO_4$, acetic acid, trifluoro acetic acid, citric acid, benzoic acid or with bases for example with amines, such as triethylamine in a known manner.

The physicochemical and spectral properties of Decaplanin are shown in Table I below:

**Table 1**

Physico-chemical and spectral properties of Decaplanin.

| | | |
|---|---|---|
| Nature | : | white powder with slightly pinkish tinge. |
| Chemical type | : | basic glycopeptide of the vancomycin class. |
| Solubility | : | Water |
| M.pt. | : | >240 °C (decomposition) |
| $[\alpha]_D$ | : | -87.6° (C 0.5, $H_2O$) |
| Thin Layer Chromatography (TLC) $R_f$ | : | 0.5 (Isopropanol:2N $NH_3$ 70:30; silica gel plate article # 5554 from E. Merck, Darmstadt). |
| High Pressure Liquid Chromatography (HPLC) $R_\tau$ | : | 3.5 mins (4 x 250 mm ODS-Hypersil (10 µ) column; eluant 15 % $CH_3CN$ in aqueous sodium phosphate buffer, pH 7.0; UV detection 220 nm, chart speed 10 mm $min^{-1}$. Flow rate 1 ml $min^{-1}$. (Figure 1 of the accompanying drawings). |
| Mol. formual | : | $C_{72}H_{86}ClN_9O_{28}$ confirmed by high-resolution FAB mass spectrometry (matrix trifluoroacetic acid/ 3-nitrobenzylalcohol measured: m/z 1560.536 as $M+H^+$ ion calculated m/z 1560.535 for $^{12}C_{72}{}^{1}H_{87}{}^{35}Cl^{14}N_9{}^{16}O_{28}$ |
| $UV\lambda_{max}$ ($H_2O$) | : | 224, 280 (302 with alkali) nm. (Figure 2 of the accompanying drawings). |

| | | |
|---|---|---|
| IR (KBr) | : | 3300 (br), 2920 (s), 1650 (s) $cm^{-1}$ (Figure 3 of the accompanying drawings). |
| $^1H$-NMR (270 MHz, $d_6$-DMSO) | : | as shown in Figure 4 of the accompanying drawings. |
| $^{13}C$-NMR (100 MHz, $H_2O/d_6$-DMSO 9:1, 343 K) | : | Figure 5, Table A |
| Amino acids residues: obtained by total acid hydrolysis | | Aspartic acid, N-methyl leucine Actinoidinic acid and a monodechloro analogue of vancomycinic acid. |
| Sugars present | : | ß-D-glucose, α-L-4-epi-vancosamine and α-L-rhamnose. |

**Table A:** Chemical Shifts of the $^{13}C$-NMR Signals of Decaplanin (ppm relative to TMS)

| | | | |
|---|---|---|---|
| 17.97 | 63.85 | 107.92 | 137.06 |
| 18.79 | 67.56 | 109.69 | 138.95 |
| 19.76 | 70.15 | 119.10 | 139.61 |
| 22.97 | 70.87 | 119.56 | 151.20 |
| 23.75 | 71.97 | 122.72 | 153.69 |
| 25.51 | 72.33 | 123.11 | 155.86 |
| 34.71 | 72.36 | 124.06 | 156.47 |
| 39.80 | 73.46 | 126.07 | 157.03 |
| 38.19 | 75.95 | 126.96 | 157.59 |
| 42.00 | 76.72 | 127.54 | 157.92 |
| 53.63 | 77.06 | 127.95 | 168.28 |
| 55.80 | 77.43 | 129.16 | 170.58 |
| 56.09 | 82.14 | 129.53 | 171.75 |
| 57.97 | 93.76 | 130.01 | 172.70 |
| 60.24 | 102.92 | 131.01 | 172.70 |
| 60.73 | 103.34 | 134.51 | 174.86 |
| 62.53 | 104.33 | 134.71 | 177.88 |
| 63.71 | 105.15 | 135.60 | 178.47 |

The structure of Decaplanin, as elucidated by complete spectroscopic analysis of the compound and its degradation products is represented in Formula IV.

## Biological activity of Decaplanin

Decaplanin displays activity against a wide range of Gram-positive bacteria including enterococci and clinical isolates which are resistant to commonly utilized antibiotics such as penicillin, methicillin, oxacillin, other β-lactam antibiotics, tetracyclines, aminoglycosides and erythromycin. The minimum inhibitory concentration (MIC) has been determined for various microorganisms and the results are shown in Table II

below:

**Table II**

MIC of Decaplanin - μg/ml

| Test Organism | Decaplanin |
|---|---|
| Staphylococcus aureus 209 P | 0.78 - 1.25 |
| S. aureus 20424 Mac$^R$ | 1.25 - 2.5 |
| S. aureus 256 E | 0.39 - 0.78 |
| S. aureus 3066 Meth$^R$ | 1.25 - 2.5 |
| S. aureus 6971 E | 0.39 - 0.78 |
| S. aureus R85 Tet$^R$ | 0.78 - 1.25 |
| S. aureus 705 | 0.39 - 0.78 |
| S. aureus R85/m Em$^R$ | 0.78 - 1.25 |
| S. aureus E 710 Meth$^R$ | 0.78 - 1.25 |
| S. aureus 712 Meth$^R$ | 1.25 - 2.5 |
| S. aureus 789 Meth$^R$ | 0.78 - 1.25 |
| S. aureus O11UC5 Mac$^R$ | 0.78 - 1.25 |
| S. aureus SG 511 | 0.78 - 1.25 |
| S. aureus 6538P Meth$^R$ | 0.78 - 1.25 |
| Staphylococcus epidermidis 178 | 0.78 - 1.56 |
| S. epidermidis 32965 | 0.78 - 1.56 |

| | |
|---|---|
| Streptococcus faecalis | 2.5 - 3.2 |
| Str. faecalis 02DODU1 Mac$^R$ | 1.25 - 2.5 |
| Str. faecalis UD8b | 0.78 - 1.25 |
| Str. feacalis 756 | 0.78 - 1.25 |
| Str. faecalis 23241 | 1.25 - 2.5 |
| Str. faecalis 21777 | 2.5 - 3.2 |
| Str. pyogenes 308 | 0.098 |
| Str. pyogenes A 77 | 0.098 |
| Str. bovis | 0.78 - 3.2 |
| Str. viridans | 0.78 - 3.2 |
| Str. pneumoniae | 0.12 - 0.39 |
| Str. haemolyticus | 1.0 - 4.0 |
| Enterococcus faecalis ATCC 29212 | 1.56 - 3.2 |
| Ent. durans 58 | 0.78 - 1.25 |
| Ent. hirae 55 | 1.56 - 2.5 |
| Ent. faecium D 59 | 2.5 - 3.2 |
| Bacillus sp. | 1.56 - 3.2 |
| Clostridium difficile | 0.39 - 1.56 |
| Clostridium perfringens | 0.12 - 0.39 |
| Corynebacterium jekeium | 0.39 - 1.25 |
| Listeria monocytogenes | 1.56 - 3.2 |
| Peptostreptococcus sp. | 0.12 - 0.39 |
| Propionibacterium acnes | 0.12 - 0.78 |

Mac = Macrolide; Meth. = Methicillin
Tet = Tetracycline; Em = Erythromycin

"In vivo" antibacterial protection studies

Decaplanin showed a good activity when tested in the in vivo system in laboratory mice experimentally infected with Staphylococcus aureus E 710 (Methicillin resistant). The results are shown in Table III.

**Table III:** Activity of Decaplanin and Vancomycin against experimental infections in Swiss mice

Inoculum : 5 x $10^9$ CFU/mouse for S. aureus E 710 (Meth$^R$)

Dosage : mg/kg x 3 (subcutaneous)

| Compound | vs. S. aureus E 710 - mg/kg | |
|---|---|---|
| | $ED_{50}$ | $ED_{90}$ |
| Decaplanin | 14.26 | 28.23 |
| Vancomycin | 18.94 | 32.01 |

**Toxicity Studies**

The acute toxicity of the compound in laboratory animals has been evaluated.

Even at doses of 1000 mg/kg and 2000 mg/kg, the highest doses so far, injected subcutaneously in Swiss mice no acute toxicity was observed. Moreover, there was an unexpected finding in that whereas with vancomycin at the same dosages tissue necrosis was seen at the site of injection, with Decaplanin no abnormalities were observed.

Acute toxicity studies done by intravenous injection of the compound into Wistar rats indicated that the $LD_{50}$ is greater than 2,500 mg/kg (i.v.).

Acute toxicity studies done by intravenous injection of the compound to Swiss white mice indicated that the $LD_{50}$ is greater than 3000 mg/kg (i.v.). Macroscopic examination on dissection of the animals at the end of the 14 day follow-up period revealed no morphological changes in either study.

These results indicate that the novel antibiotic of this invention, namely Decaplanin, is very well tolerated and may be adminstered by the oral, intramuscular or intravenous routes.

Thus the invention also relates to medicinal products, for the treatment of microbial infections, which contain the compound Decaplanin.

The compound according to the invention can also be used in combination with other active substances, for example from the series of penicillins, cephalosporins, aminoglycosides or quinolones.

Decaplanin and its physiologically tolerated acid addition salts can be administered, for example, orally, intramuscularly or intravenously. Medicinal products which contain Decaplanin as active substance can be prepared by mixing the said compound with one or more pharmacologically tolerated vehicles of diluents such as, for example, fillers, emulsifiers, lubricants, masking flavours, colorants or buffer substances, and converted into a suitable pharmaceutical form such as, for example, tablets, coated tablets, capsules, or a suspension or solution suitable for parenteral administration.

Examples of vehicles or diluents which may be mentioned are tragacenth, lactose, talc, agar.agar, polyglycols, ethanol and water. Suitable and preferred for parenteral administraiton are suspension or solutions in water. It is also possible to administer the active substances as such, without vehicles or diluents, in a suitable form, for example in capsules.

Suitable doses of the compound of this invention or its physiologically tolerated acid addition salts are about 0.4 to 20 g/day, preferably 0.5 to 4 g/day, for an adult of body weight about 60 kg.

It is possible to administer single doses or, in general, multiple doses, it being possible for the single dose to contain the active substance in an amount of about 50 to 4,000 mg, preferably of about 500 to 2,000 mg.

The following examples are illustrative but not limitative of the scope of the present invention:

**Example 1**

Isolation of actinomycete culture No. Y-86,36910 from soil

a) Preparation of the isolation nutrient media

| | | |
|---|---|---|
| Medium 1: | Glucose | 1.0 g |
| | Glycerol | 1.0 g |
| | L-Arginine | 0.3 g |
| | $K_2HPO_4$ | 0.3 g |
| | $MgSO_4.7H_2O$ | 0.2 g |
| | NaCl | 0.3 g |
| | Yeast extract | 2.0 g |
| | $Fe_2(SO_4)_3$ | 10.0 mg |
| | $CuSO_4.5H_2O$ | 1.0 mg |
| | $ZnSO_4.7H_2O$ | 1.0 mg |
| | $MnSO_4.7H_2O$ | 1.0 mg |
| | Agar | 15.0 g |
| | Distilled water | 1 litre |
| | pH | 6.5 |

| | | |
|---|---|---|
| Medium 2: | Glucose | 2.0 g |
| | L-Asparagine | 1.0 g |
| | $K_2HPO_4$ | 0.5 g |
| | $MgSO_4.7H_2O$ | 0.5 g |
| | Soil extract | 200 ml |
| | Agar | 15.0 g |
| | Distilled water | 800 ml |
| | pH | 8.0 |

| | | |
|---|---|---|
| Medium 3: | Starch | 10.0 g |
| | Casein | 0.3 g |
| | $KNO_3$ | 2.0 g |
| | NaCl | 2.0 g |
| | $K_2HPO_4$ | 2.0 g |
| | $MgSO_4.7H_2O$ | 0.05 g |
| | $CaCO_3$ | 0.02 g |
| | $FeSO_4$ | 0.01 g |
| | Agar | 15.0 g |
| | Distilled water | 1 litre |
| | pH | 7.2-7.5 |

The media were sterilised at 121 °C for 1/2 hour.

b) Preparation of the soil suspension

A gram of soil is suspended in distilled water, shaking vigorously. The soil is allowed to settle out, and the supernatant liquid is used to inoculate each of the abovementioned isolation media.

c) Inoculation of the isolation medium

Petri dishes containing 50 ml of one of the abovementioned isolation nutrient media are each inoculated with 1 ml of soil suspension.

d) Isolation of actinomycete culture No. Y-86,36910

The inoculated petri dish is incubated at 30 °C for two weeks and actinomycete culture No. Y-86,36910 is isolated from the growing microorganisms.

**Example 2**

Culturing of actinomycete culture No. Y-86,36910 for the preparation of Decaplanin by fermentation.
Actonomycete culture No. Y-86,36910 is maintained on yeast-malt agar of the following compositon:

| | |
|---|---|
| Malt extract | 10.0 g |
| Yeast extract | 4.0 g |
| Glucose | 4.0 g |
| Agar | 15.0 g |
| Distilled water | 1 litre |
| pH | 7.0 |

The medium is distributed over test tubes and sterilized at 121 °C for 30 minutes. To prepare agar slants, the tubes are cooled in an inclined position. Each agar slant is inoculated with the culture and incubated at 28 °C for 10-15 days, by which time good growth and spore formation is observed. A distilled water suspension of spores on the vegetative mycelium from an agar slant is used to inoculate 5 x 500 ml Erlenmeyer flasks each containing 100 ml of seed culture medium.

| | |
|---|---|
| Glucose | 15.0 g |
| Soybean meal | 15.0 g |
| Cornsteep Liquor | 5.0 g |
| $CaCO_3$ | 2.0 g |
| NaCl | 5.0 g |
| Distilled water | 1 litre |
| pH | 6.5 |

The above medium is distributed in 100 ml portions in 500 ml Elenmeyer flasks and sterilized for 30 minutes at 121 °C. The flasks are cooled, inoculated with the culture and shaken at 29 °C (± 1 °C) on a rotary shaker with a 38 mm throw at 240 rpm for 72 hours. The product which has grown is used to inoculate two 15 l glass fermentation vessels, each containing 10 l, with 8-10 % (by volume) seed culture medium, for the preparation of the second seed culture stage. The fermentation is carried out at 29 °C (± 1 °C), stirring at 180-200 rpm and aerating at 6-7 litres/min, for a period of 48 hours.

The resulting, well-grown second seed culture stage is used for inoculation of the production medium.

Composition of the production medium:

| | |
|---|---|
| Dextrin | 20.0 g |
| Soybean flour | 10.0 g |
| Yeast extract | 1.0 g |
| $FeSO_4.7H_2O$ | 0.1 g |
| Distilled water | 1 litre |
| pH | 7.0 |

0.025 % Desmophen® is added to the contents of the fermentation vessel as antifoam agent.

280 l of the above medium is placed in a 390 l fermentation vessel and sterilised by indirect and direct steam at 121 °C for 28 minutes. The fermentation vessel is cooled and inoculated with the second seed culture stage (8 % by volume). The fermentation is carried out at 29 °C (± 1 °C), stirring at 100-120 rpm, for a period of 66-68 hours. The aeration rate is 170 litres per minute. When the fermentation is terminated after 66-68 hours, the diameter of the zone of inhibition of Staphylococcus aureus 209 P is 16 mm when the culture filtrate is tested by the agar well method (6 mm diameter), with the pH of the culture liquid being in the range 6.5 to 7.0. The packed cell volume is ca. 20 %.

The harvested culture broth which contains the antibiotic complex is centrifuged to remove the mycelium, and the culture liquid is further processed as described in Example 4.

**Example 3**

Cultivation of actinomycete culture No. Y-86,36910 for the preparation of Decaplanin by fermentation.

The process is that of Example 2, with the following differences:

Composition of the production medium:

| | |
|---|---|
| Starch | 20.0 g |
| Soybean meal | 10.0 g |
| $CaCO_3$ | 3.0 g |
| NaCl | 5.0 g |
| $FeSO_4.7H_2O$ | 0.1 g |
| Distilled water | 1 litre |
| pH | 7.0 |

100 l of the above medium are introduced into a 150 l fermentation vessel and sterilized by direct and indirect steam at 121 °C for 28 minutes. The fermentation vessel is cooled and inoculated with the second seed culture stage (8 % by volume). The fermentation is carried out at 29 °C (± 1 °C), stirring at 100-120 rpm, with an aeration rate of 70-90 litres per minute. When the fermentation is terminated after 66-68 hours, the pH of the culture broth is 7.0 and the diameter of the zone of inhibition of Staphylococcus aureus 209 P is 17 mm when the culture filtrate is tested by the agar well method (6 mm diameter). The packed cell volume is 20 %. The culture broth is processed as in Example 4.

**Example 4**

Isolation and purification of Decaplanin

Approximately 645 l of the harvested broth, as obtained in Examples 2 and 3, was separated from the mycelium by centrifugation. The resulting broth filtrate (pH 6.5-7.0) was passed through columns of a total of 25 l of Diaion® HP-20. The columns were thoroughly washed with a total of 500 l demineralized water and then with 100 l of 30 % methanol in water. Decaplanin was eluted out of the columns with a total of 200 l of 70 % methanol in water, 100 l methanol and 200 l of 50 % acetone in water and the bioactivity was monitored by testing the eluates against Staphylococcus aureus 209 P, S. aureus 20240 and Micrococcus luteus. The active eluates were combined and then concnetrated under reduced pressure to remove the organic solvents.

The resulting aqueous solution (180 1) containing Decaplanin was decolourized by passing through a column of 5 l of Amberlite® IRA 68 ($Cl^-$) set in demineralized water. The column was washed with 10 l of demineralised water. The effluents and the water washings were combined and passed through a column of 10 l of Diaion® HP-20 in water. The column was washed with 40 l of demineralized water till the water washings were free of chloride ions. The column was then eluted with 70 l of 50 % acetone in water and the active eluates were concentrated in vacuo to remove acetone and most of the water. The resultant 700 ml

of the aqueous solution was lyophilized to give 15 g of semi-pure Decaplanin as a slightly yellowish powder.

The semi-pure Decaplanin thus obtained was subjected to medium pressure liquid chromatography (MPLC) in a 5.5 x 53 cm Labomatic® glass column packed with dimethyloctadecyl silylated silica gel (RP 18). The column was initially set in a 0.1 M solution of $NH_4OAc$ in demineralized water and then eluted with mixtures of $CH_3CN$ with the aqueous $NH_4OAc$ solution, the amount of $CH_3CN$ being 2 % (1.2 l), 4 % (5.6 l), 6 % (2.4 l), 10 % (2.4 l) and 15 % (2.8 l). The flow rate was maintained at 30 ml $min^{-1}$ and the eluates collected in fractions of 500 ml were monitored by UV detection at 220 nm as well as by microbial bioassay. Decaplanin was eluted from the column by 2 % and 4 % $CH_3CN$ in the aqueous $NH_4OAc$ solution. The eluates were combined and concentrated under reduced pressure to remove the $CH_3CN$.

The resulting aqueous solution of Decaplanin was passeed through a column of 600 ml of Diaion® HP-20 set in double distilled water. The column was washed with 2 l of double distilled water and then eluted with 4 l of 50 % acetone in water. The active eluates were concentrated under reduced pressure to remove acetone and then lyophilized to afford 8.4 g of Decaplanin as a white powder with a slightly pinkish tinge.

**Example 5**

Preparation of Decaplanin salts; and their reconversation to the free base.

Decaplanin, a basic compound, can be converted to its acid addition salts by treatment with strong acids, e.g. HCl, $H_2SO_4$, $CF_3COOH$, $CH_3COOH$.

To 100 mg Decaplanin (free base) dissolved in 10 ml distilled water was added 64 µl of 1N HCl at 0 °C followed by stirring at room temperature for 3 hours, then lyophilization to produce 95 mg of the hydrochloride salt. Chlorine analysis of this Decaplanin•HCl salt shows to be a monohydrochloride.

Decaplanin salts may be reconverted to the free base by treatment with anion exchange resins.

Decaplanin•$CF_3COOH$ salt (200 mg) in 200 ml distilled water is treated with 5 g of Dowex 12 x ($OAc^-$) in water, with stirring for 30 mins at room temperature then filtered. The filtrate is lyophilized to give 140 mg of the Decaplanin free base.

**Claims**

1. Decaplanin, a compound of the formula

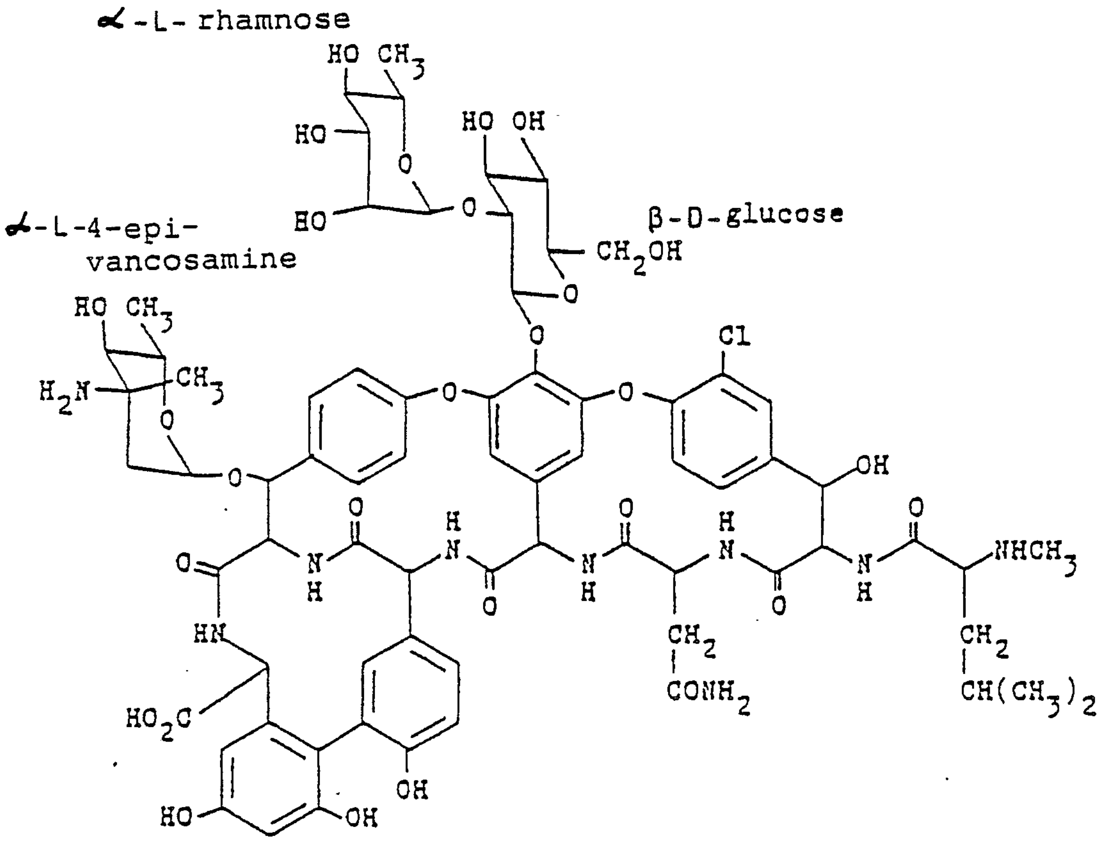

as well as obvious chemical equivalents and physiologically acceptable salts thereof.

2. A process for the preparation of the compound as claimed in claim 1, which comprises cultivation of the actinomycete culture Y-86,36910 (DSM 4763) under aerobic conditions in an aqueous nutrient medium which contains carbon sources, nitrogen sources, inorganic nutrient sources and mineral salts.

3. A process as claimed in claim 2, wherein the cultivation is carried out at a temperature between 18 and 40 °C.

4. A process as claimed in claim 2 or 3, wherein the cultivation is carried out at a pH between 5.5 and 8.5.

5. A process as claimed in one or more of claims 2-4, wherein the cultivation is carried out at a temperature of 29 °C (± 1 °C) and at a pH of about 6.5.

6. A process as claimed in one or more of claims 2-5, wherein the cultivation is carried out longer than 66 hours.

7. A process as claimed in one or more of claims 2-6, wherein the cultivation is carried out as a submerged cultivation.

8. A pharmaceutical which contains a compound as claimed in claim 1, if appropriate together with customary auxiliaries and/or vehicles.

9. The use of a compound as claimed in claim 1 for the preparation of pharmaceuticals having an antibiotic action.

10. Actinomycete culture Y-86,36910 (DSM 4763).

Claims for the following Contracting States: ES, GR

1. A process for the preparation of Decaplanin, a compound of the formula

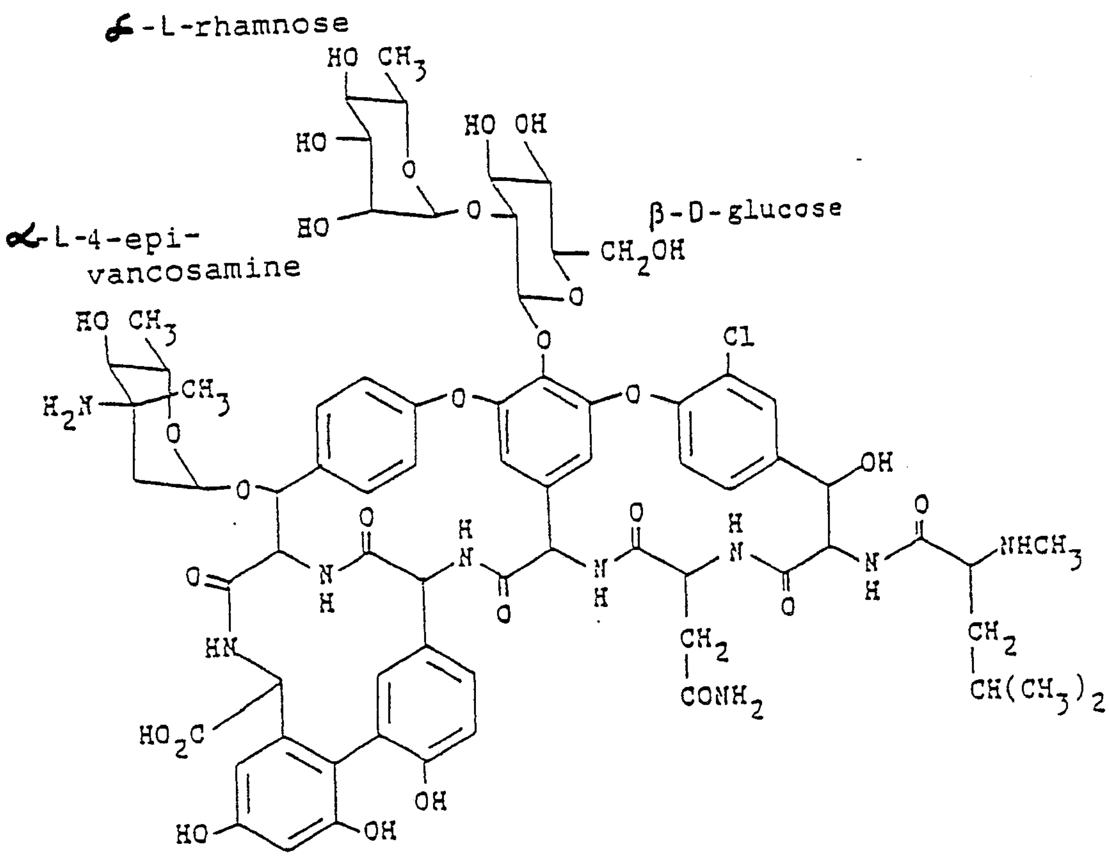

which comprises cultivation of the actinomycete culture Y-86,36910 (DSM 4763) under aerobic conditions in an aqueous nutrient medium which contains carbon sources, nitrogen sources, inorganic nutrient sources and mineral salts.

2. A process as claimed in claim 1, wherein the cultivation is carried out at a temperature between 18 and 40 °C.

3. A process as claimed in claims 1 or 2, wherein the cultivation is carried out at a pH between 5.5 and 8.5.

4. A process as claimed in one or more of claims 1-3, wherein the cultivation is carried out at a temperature of 29 °C (± 1 °C) and at a pH of about 6.5.

5. A process as claimed in one or more of claims 1-4, wherein the cultivation is carried out longer than 66 hours.

6. A process as claimed in one or more of claims 1-5, wherein the cultivation is carried out as a submerged cultivation.

7. A process for the production of a pharmaceutical wherein a compound which is produced as claimed in one or more of claims 1-6, is together with customary auxiliaries and/or vehicles transformed into a form suitable for administration.

8. The use of a compound as claimed in claim 1 for the preparation of pharmaceuticals having an antibiotic action.

9. Actinomycete culture Y-86,36910 (DSM 4763).

FIG.1 HPLC CHROMATOGRAM OF DECAPLANIN

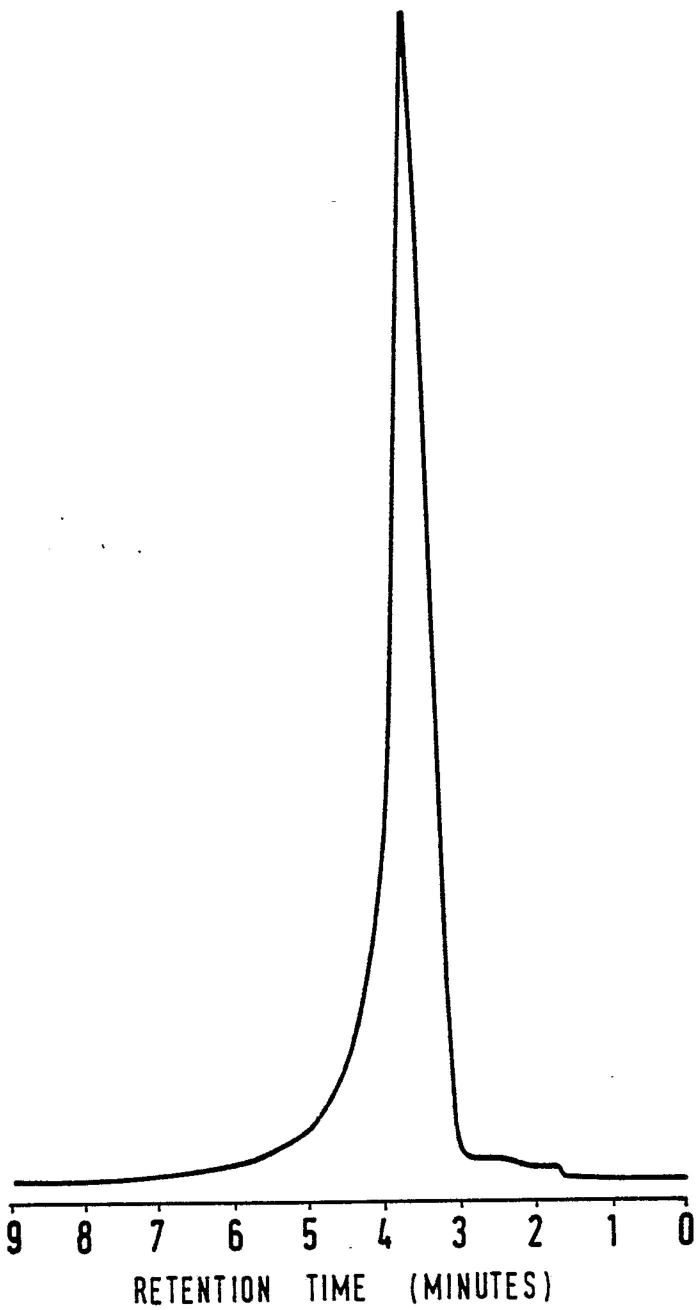

FIG. 2

UV SPECTRUM OF DECAPLANIN

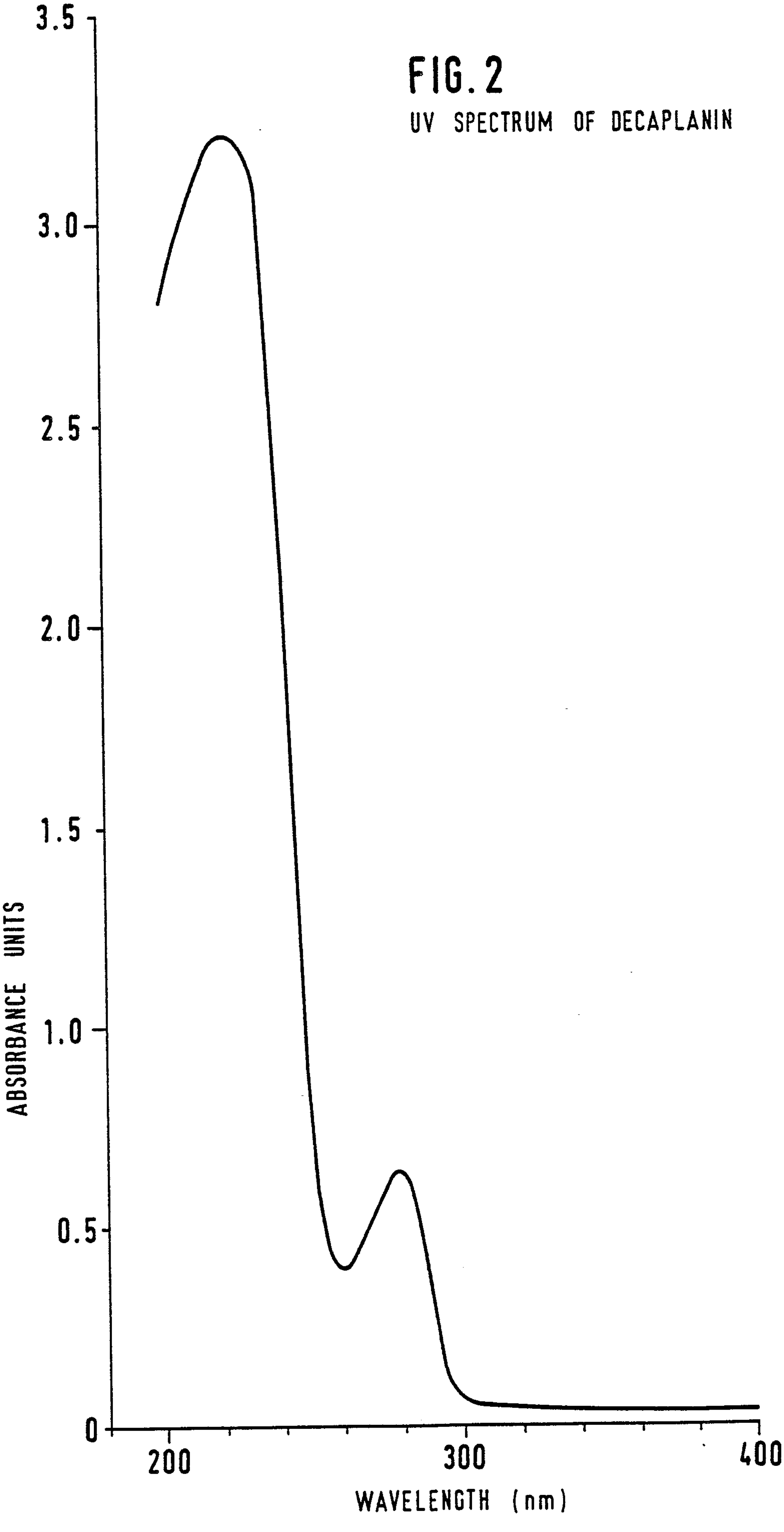

FIG.3 IR SPECTRUM OF DECAPLANIN

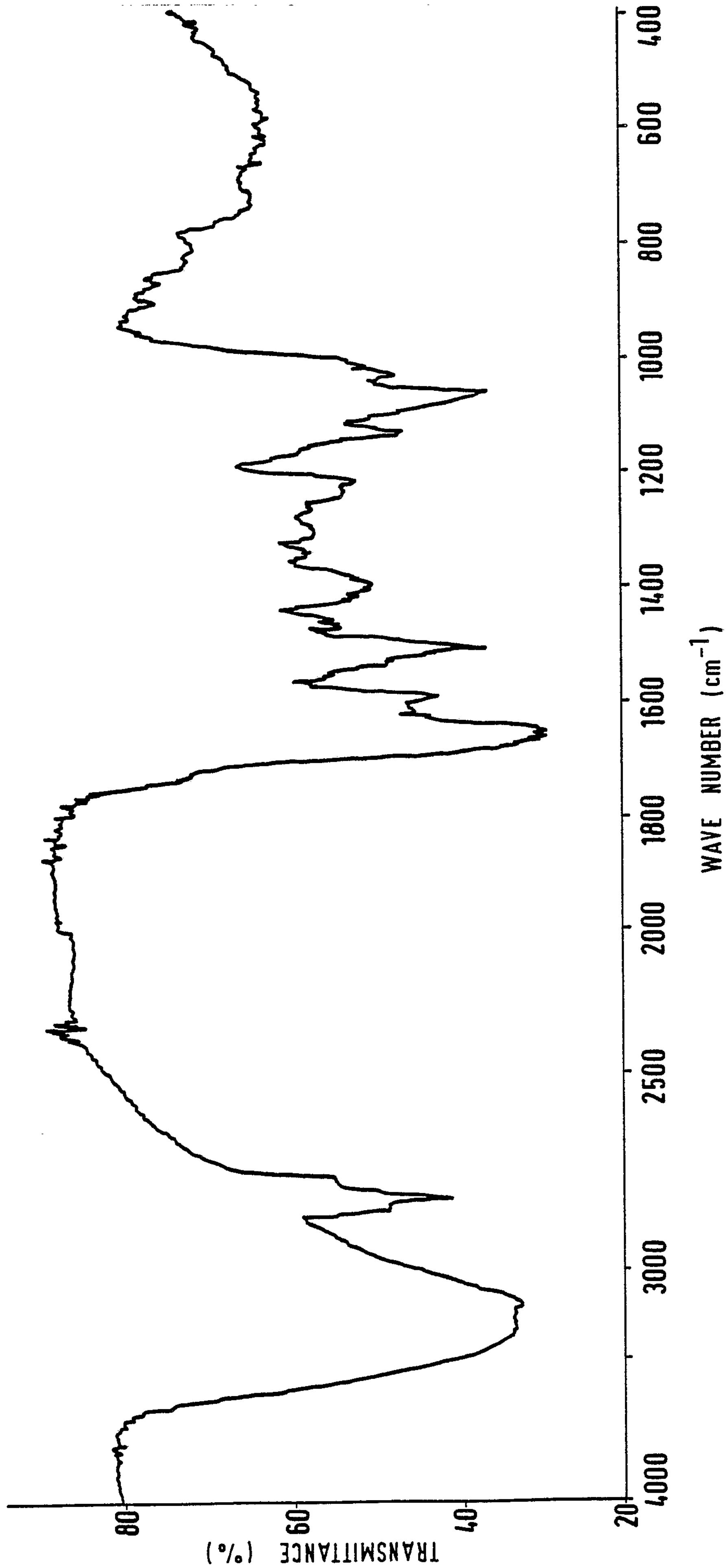

FIG.4 $^1H$-NMR OF DECAPLANIN

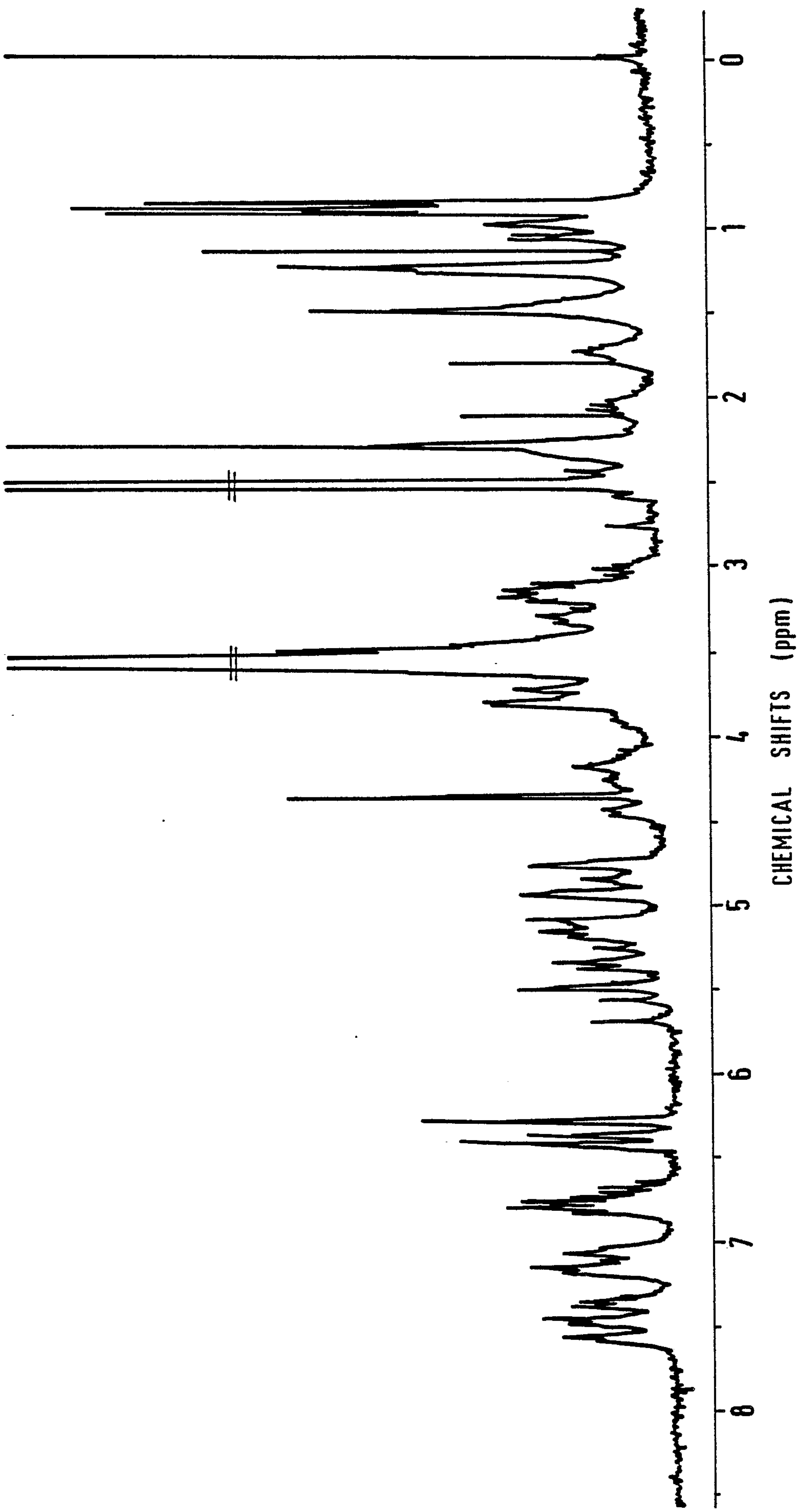

FIG. 5 $^{13}C$-NMR-SPECTRUM OF DECAPLANIN

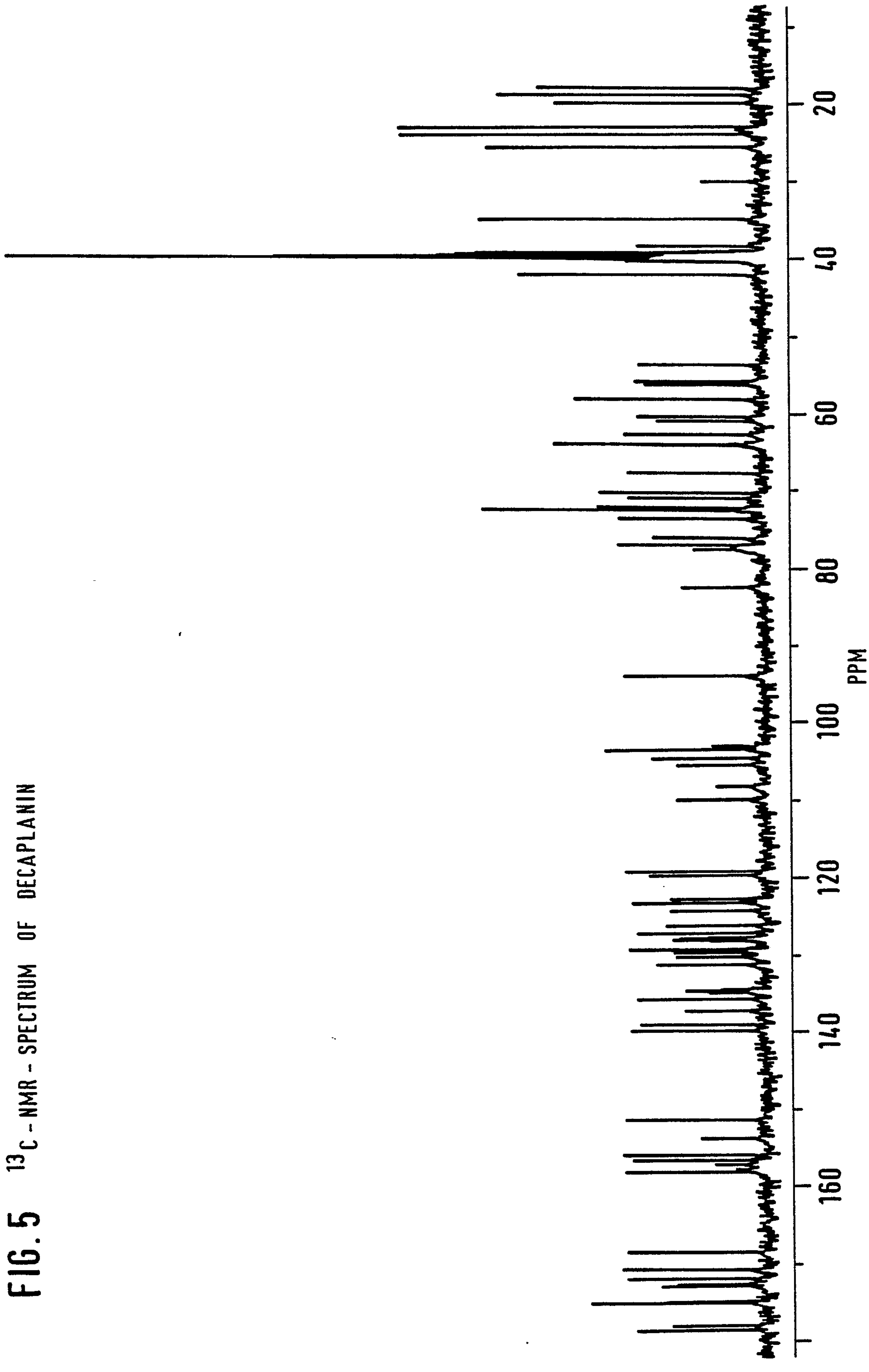

European Patent Office

# EUROPEAN SEARCH REPORT

Application Number

EP 89 11 5519

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | THE JOURNAL OF ANTIBIOTICS, vol. 42, no. 4, April 1989, pages 497-505, Japan Antibiotics Research Association, Tokyo, JP; E. RIVA et al.: "A42867, a novel glycopeptide antibiotic"<br>--- | | C 07 K 9/00<br>C 12 P 21/04<br>A 61 K 37/02 //<br>(C 12 P 21/04<br>C 12 R 1:04 ) |
| A | EP-A-0 231 111 (SHIONOGI SEIYAKU K.K.)<br>----- | | |
| | | | TECHNICAL FIELDS SEARCHED (Int. Cl.5) |
| | | | C 07 K<br>C 12 P<br>A 61 K |

The present search report has been drawn up for all claims

| Place of search | Date of completion of the search | Examiner |
|---|---|---|
| THE HAGUE | 23-11-1989 | NOVOA Y SANJURJO M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document